(19) Europäisches Patentamt / European Patent Office / Office européen des brevets

(11) **EP 4 385 526 A1**

(12) **EUROPEAN PATENT APPLICATION**

(43) Date of publication:
**19.06.2024 Bulletin 2024/25**

(21) Application number: **23213284.5**

(22) Date of filing: **30.11.2023**

(51) International Patent Classification (IPC):
**A61K 51/12** (2006.01)   **A61P 35/00** (2006.01)
**A61K 39/00** (2006.01)   **C12N 5/0783** (2010.01)

(52) Cooperative Patent Classification (CPC):
**A61K 39/4611; A61K 39/4631; A61K 51/1203;
A61P 35/00; C12N 5/0636;** C12N 2521/10

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **02.12.2022 DE 102022132082**

(71) Applicant: **Horia Hulubei National Institute for R &
D in**
**Physics and Nuclear Engineering (IFIN-HH)
077125 Magurele (RO)**

(72) Inventor: **Spohr, Klaus, Dr.
Paisley, Scotland, PA2 0TX (GB)**

(74) Representative: **Ostertag & Partner Patentanwälte
mbB
Azenbergstraße 35
70174 Stuttgart (DE)**

(54) **METHOD FOR LOADING IMMUNOCOMPETENT CELLS WITH NANOPARTICLES AND/OR A CYTOTOXIC SUBSTANCE AND IMMUNOCOMPETENT CELLS FOR USE IN THERANOSTIC TREATMENT**

(57) The present invention provides a method for loading immunocompetent cells with nanoparticles and/or a cytotoxic substance for use in the theranostic treatment of patient-specific genetically modified cells. The method comprises the steps: (a) providing immunocompetent cells; and (b) loading the immunocompetent cells with the nanoparticles and/or the cytotoxic substance, wherein the nanoparticles and/or the cytotoxic substance are nuclides for radiotherapy. The loading step comprises (i) applying the nanoparticles and/or the cytotoxic substance to the immunocompetent cells or mixing the nanoparticles and/or the cytotoxic substance with the immunocompetent cells in a liquid carrier; and (ii) poration of the immunocompetent cells in order to increase the permeability of the cell membranes of the immunocompetent cells for the nanoparticles and/or the cytotoxic substance and thereby loading the immunocompetent cells with the nanoparticles and/or the cytotoxic substance.

← 5 - 7 μm →

Fig. 1

EP 4 385 526 A1

**Description**

The invention relates to a method for the loading of immunocompetent cells, especially of genetically transfected immunocompetent cells, with nanoparticles and/or a cytotoxic substance for the theranostic treatment of patient-specific genetically modified cells. In particular, these cells comprise viral genetically modified cells or patient-specific malignant tumors in an affected patient. The invention further relates to immunocompetent cells, immunocompetent cells for use in a method for the theranostic treatment of patient-specific genetically modified cells and a medical composition.

T cell therapy is a novel and proven method for the treatment of genetically modified cells, in particular viral genetically modified cells, including most recently Covid-19, or a patient-specific malignant tumor. Recent studies highlight the unique potential for new $\gamma\delta$-CAR T cell-based treatments (Mirzaei et al. (2016): "Prospects for chimeric antigen receptor (CAR) T cells: A potential game changer for adoptive T cell cancer immunotherapy"). These special cells have a specific T-cell receptor (TCR) on their surface. In their natural form, $\gamma\delta$ T cells make up 2% to 10% of peripheral blood T lymphocytes. Among others, techniques developed for $\alpha\beta$ T-cell therapies can also be applied.

A decisive advantage of $\gamma\delta$ T cells over $\alpha\beta$ T cells is the innate tumor recognition ability of $\gamma\delta$ T cells, which enables the targeted killing of cancerous tissues. If genetically modified $\gamma\delta$ CART cells are used, in which the specificity towards patient-specific genetically modified cells has been increased by a chimeric anti-gene receptor (CAR), the side effects of treatment can be kept to a minimum.

The particular cancer selectivity of genetically modified $\gamma\delta$ CAR T cells is based on the ability of the chimeric antigen cell receptor (CAR) introduced into the cells by genetic engineering to recognize the isopentenyl pyrophosphate (IPP) antigen located on the surface of the cancer cell due to a disturbed isoprenoid signaling pathway in the body of the affected cell (Zeng, Jieming et al. (May 2019): "Derivation of mimetic T cells endowed with cancer recognition receptors from reprogrammed T cell").

In response to the recognition of a cancer cell, the attacking $\gamma\delta$ CAR-T cells activate inflammatory cytotoxic substances, which they inject into the malignant cell via the so-called "kiss-of-death" mechanism (Rozenbaum, Meir et al. (July 2020): "Gamma-Delta CAR-T Cells Show CAR-Directed and Independent Activity Against Leukemia"). In-vitro and clinical phase trials with $\gamma\delta$-T cells have already shown promising results and are increasingly establishing themselves in the treatment of leukemia, neuroblastoma, various carcinomas, and most recently, Covid-19 (Caron et al. (Mar. 2021): "How to Train Your Dragon: Harnessing Gamma Delta T Cells Antiviral Functions and Trained Immunity in a Pandemic Era"). As the field of immunotherapy moves beyond the repeated iteration of established methods to more creative solutions, $\gamma\delta$ CAR-T cells offer the opportunity to advance established cancer treatments and break new ground in oncology. These new methods are broad-based and have a much more favorable safety profile than conventional therapies, as the surrounding healthy cell tissue is spared during treatment due to the specificity of $\gamma\delta$ CAR-T cells.

In conventional therapy with $\alpha\beta$ CAR-T cells, for example, two signals that stimulate the T cells are triggered by activating the chimeric antigen receptor (CAR) attached to the outside of the T cell. A disadvantage of $\alpha\beta$ CAR-T cells is that they can also bind to healthy cells, which leads to the death of healthy cells after activation of the T cells. This phenomenon is referred to as "off-tumor toxicity" and causes strong side effects, such as those observed with classic chemotherapy.

The $\gamma\delta$ CAR-T cells differ from other T cells in that they have a specific $\gamma\delta$ T cell receptor (TCR) that only binds to antigens that are characteristic of impaired unhealthy cells. The TCR is a safety barrier against activation of the T cells in healthy cells. A genetically modified $\gamma\delta$ CAR-T cell, which only has one co-stimulatory domain, only binds to one damaged cell. This reduces side effects, as healthy cells are not attacked. To obtain sufficient cell material, work is currently being carried out on allogeneic cell pools for which $\gamma\delta$ T cells are taken from healthy donors, multiplied, and distributed worldwide via a distribution system.

The potential of $\gamma\delta$ T cells is further enhanced by the recently developed method, which makes it possible to switch from individual autologous therapy (see US 9,855,298 B2), which is associated with a wide range of side effects, to allogeneic therapy.

The allogeneic use of $\gamma\delta$ T cells reduces graft-versus-host disease (GvHD), even if this initially appears to be a contradiction in terms. This is possible because the current $\gamma\delta$ CAR-T cell technology allows us to overcome the restriction triggered by the major histocompatibility complex (MHC). Therefore, the risk of GvHD is minimized (Handgretinger et al. (Mar. 2018): "The potential role of T cells after allogeneic HCT for leukemia"). In addition, various studies have shown overall favorable outcomes of $\gamma\delta$ CAR-T cell-based treatments.

The effect of a high $\gamma\delta$ CAR-T cell immune reconstitution has been recorded, e.g., after hematopoietic stem cell transplantation (HSCT). In concomitant clinical studies, patients with an increased number of $\gamma\delta$ CAR-T cells had a significantly higher overall survival rate and undetectable acute GvHD manifestations.

In addition, the likelihood of transplant rejection in patients with severe viral infections such as HIV, influenza, and COVID-19 is low, and corresponding advanced clinical trials have been ongoing since early 2022.

It is known that solid tumors are subject to further mutations over time, which also change the structure of the externally accessible receptors, which can currently only be recognized after a histological or histo-pathological examination, followed by more detailed examination methods.

Therapeutic approaches with immunocompetent cells can be found, for example, in WO 2022241151 A2, WO 2022238962 A1, WO 2022241036 A1, WO 2022147014 A2, and WO 2020109953 A1. Further therapeutic approaches can be found in WO 2019 181018 A1, WO 9817342 A2, CN 115215851 A, CN 115232129 A, FR 3120630 A1, CN 112675442 A, and JP 2016159107 A.

The disadvantage of previous treatment methods against patient-specific genetically modified cells, particularly against cancer, is that classic chemotherapy substances are administered that have a good toxic effect on the cancer cells but also kill healthy cells in a completely undirected manner. This often results in massive side effects and generally poor prospects of successful treatment because the administered substances do not reach the affected, particularly tumorous, tissue in a targeted manner.

Even those therapies that use $\alpha\beta$ T cells transfected with a gene coding for a chimeric antigen receptor (CAR) do not offer the desired specificity of treatment, as the CAR also binds to surface proteins of cells that are not genetically modified, i.e. healthy. Although fewer side effects are observed with these approaches than with conventional chemotherapy, they do not offer the desired specificity and have been proven to be insufficient. In addition, these therapies only allow the progress of the therapy to be controlled and monitored in a roundabout way. Another disadvantage is that even if the therapy is successful, there is a high risk that cells of an affected tissue will survive, and the genetically modified cells will divide again. This can then trigger new viral infections or lead to a renewed flare-up of the infection and, in the case of tumor tissue, to (further) metastases.

In view of these disadvantages of prior art therapeutic approaches using immunocompetent cells like T cells the present invention has the object to improve such therapeutic approaches.

This object is solved by a method for loading immunocompetent cells with nanoparticles and/or a cytotoxic substance as it is described in claim 1. Preferred embodiments of the claimed method are outlined in the dependent claims. Furthermore, this object is solved by correspondingly loaded immunocompetent cells and by a medical composition comprising these loaded immunocompetent cells according to the further independent claims and, in preferred embodiments, in the further dependent claims.

Moreover, the object is solved by a method for preparation of genetically transfected immunocompetent cells loaded with nanoparticles and/or a cytotoxic substance, by correspondingly prepared immunocompetent cells and by a corresponding medical composition according to the described further aspects of the invention.

The method according to the invention is characterized by loading immunocompetent cells with nanoparticles and/or a cytotoxic substance for use in the theranostic treatment of patient-specific genetically modified cells, e.g. tumor cells or other viral genetically modified cells. The method comprises the following steps:

a. Providing immunocompetent cells;

b. loading the immunocompetent cells with the nanoparticles and/or the cytotoxic substance, comprising

i. applying the nanoparticles and/or the cytotoxic substance to the immunocompetent cells or mixing the nanoparticles and/or the cytotoxic substance with the immunocompetent cells in a liquid carrier;

ii. Porating of the immunocompetent cells in order to increase the permeability of the cell membranes of the immunocompetent cells for the nanoparticles and/or the cytotoxic substance and thereby loading the immunocompetent cells with the nanoparticles and/or the cytotoxic substance.

The invention provides a method for the production of immunocompetent cells, especially genetically transfected immunocompetent cells, wherein the immunocompetent cells are loaded with nanoparticles and/or a cytotoxic substance, resulting in improved immunocompetent cells for theranostic treatment approaches. The theranostic treatment is directed against cells of the patient which are patient-specific genetically modified, in particular viral genetically modified cells or other genetically modified cells like a patient-specific malignant tumor in an affected patient. The invention makes it possible to produce immunocompetent cells that counteract the disadvantages mentioned in the prior art and have higher immunocompetence than current patient-specific genetically modified cells. The loaded immunocompetent cells according to the invention have improved toxicity, and the treatment with them promises a higher chance of success.

Accordingly, the main point of the invention is that the immunocompetent cells, which interact specifically with certain altered cells of a patient, for example tumor cells, and can combat them, are loaded with nanoparticles and/or cytotoxic substances, wherein the nanoparticles and/or the cytotoxic substance are nuclides for radiotherapy. This loading of the immunocompetent cells can, on the one hand, considerably increase the efficiency in combating the altered cells. On the other hand, this approach according to the invention allows a controlled and controllable treatment of the patient, as described below in connection with the particularly preferred embodiments of the invention.

The term "nuclides for radiotherapy" comprises stable isotopes as well as radioisotopes which are useful for radiotherapy and nuclear medicine as will be outlined in more detail below.

In especially preferred embodiments the provided immunocompetent cells have been transfected with at least one gene whose gene product has a specificity for the immunocompetent cells to the patient-specific genetically modified cells.

In preferred embodiments the loading step b. comprises:

i. Application of the nanoparticles and/or the cytotoxic substance to the immunocompetent cells or mixing of the nanoparticles and/or the cytotoxic substance with the immunocompetent cells in a carrier liquid to form a cell solution;

ii. Poration, in particular by sonoporation, electroporation, or laser poration, of the immunocompetent cells to increase the permeability of the cell membranes of the immunocompetent cells for the nanoparticles and/or the cytotoxic agent to enter. Thereby, the immunocompetent cells are loaded with the nanoparticles and/or the cytoxic substances.

The invention is based on the finding that genetically modified immunocompetent cells, whose specificity with respect to patient-specific genetically modified cells has been increased and which already produce their own, i.e. native, cytotoxic substances and inject them into the patient-specific genetically modified cells, can be further improved by addition and loading of further substances in the form of nanoparticles and/or cytotoxic substances. The nanoparticles and/or cytotoxic substances have a direct or indirect cytotoxic effect on the patient-specific genetically modified cells. This increases the probability of successfully discharging the nanoparticles and/or the cytotoxic substance from the immunocompetent cells into the patient-specific genetically modified cells, which can also lead to the death of the corresponding cells.

The nanoparticles and/or the cytotoxic substances are nuclides for radiotherapy. Preferably, the nanoparticles and/or the cytotoxic substance is at least one stable isotope and/or at least one radioisotope from the following group of stable isotopes or radioisotopes: $^{10}$B, $^{11}$B, $^{nat}$B, $^{157}$Gd, $^{99m}$Tc, $^{177}$Lu, $^{18}$F, and $^{211}$At or mixtures thereof. $^{10}$B and/or $^{157}$Gd are especially preferred.

In especially preferred embodiments the nuclides are intented to be activated later by supportive radiotherapy, e.g. in connection with boron neutron capture therapy (BNCT).

It is very advantageous if the poration is carried out under very mild and preferably controlled conditions, as the immunocompetent cells are very sensitive.

Preferably, the poration is carried out by sonoporation or electroporation or laser poration. Sonoporation is especially preferred.

In especially preferred embodiments the loading of the immunocompetent cells with nanoparticles and/or a cytotoxic substance is performed in the presence of microbubbles (also called microsomes), like liposomes. When performing the poration and especially the sonoporation in the presence of microbubbles, the loading is especially effective. Preferably the diameter of the microbubbles is 0.5 $\mu$m to 4 $\mu$m, preferably 1 $\mu$m to 3 $\mu$m, more preferably about 2 $\mu$m.

Preferably, the poration step is a sonoporation step and

a) there are free microbubbles in the cell solution and/or

b) the nanoparticles or the cytotoxic substance are coupled to microbubbles prior to the sonoporation step, wherein the nanoparticles or the cytotoxic substance being coupled to the microbubbles in such a way that, after coupling, they are located in an interior space of the microbubbles in a microbubble membrane, or they are embedded or covalently bound to the microbubble membrane from the outside.

A further advantage arises if the step of coupling the nanoparticles and/or the cytotoxic substance with the microbubbles is carried out before or after the nanoparticles or the cytotoxic substance is applied to or mixed with the microbubbles together with the immunocompetent cells.

It is also favorable if the diameter of the microbubbles is 0.5 $\mu$m to 4 $\mu$m, preferably 1 $\mu$m to 3 $\mu$m, preferably about 2 $\mu$m.

The term "immunocompetent cells" comprises different cells which may be naturally occurring or artificially designed.

In especially preferred embodiments the immunocompetent cells are T cells., and especially $\gamma\delta$ T cells are preferred.

Moreover, it is principally possible to use other immunocompetent cells, even artificial cells without DNA, which have specific receptors comparable to T cells.

In preferred embodiments the immunocompetent cells are genetically transfected cells that have been transfected with at least one gene whose gene product confers specificity for the immunocompetent cells with respect to the patient-specific genetically modified cells.

It is advantageous when the immunocompetent cells comprise at least one gene for the specificity of the immunocompetent cells in view of the patient-specific genetically modified cells. Preferably, the immunocompetent cells have been transfected with a set of genes encoding at least a portion of a chimeric antigen receptor (CAR), wherein the CAR comprises at least an extracellular specificity-mediating scFv antibody domain, an intracellular T cell activation domain, and a protein in the cell membrane connecting the scFv antibody domain and the T cell activation domain and causing the protein to be expressed in the cell membrane with the specificity-mediating gene coding for the scFv antibody domain.

Advantageously, the immunocompetent cells are CAR-T cells, preferably $\gamma\delta$ CAR-T cells.

Allogeneic immunocompetent cells are especially preferred, in particular allogeneic $\gamma\delta$ T cells, preferably allo-geneic $\gamma\delta$ CAR T cells.

Preferably, in order to control the poration step, in particular to control the permeabilization of the cell membranes, a stationary or a non-stationary electric or electromagnetic field is applied to the cell solution, especially in connection with sonoporation. This measure is carried out in particular to control and monitor the resealing or healing of the cell membranes after poration.

In order to provide a sufficient amount of loaded immunocompetent cells for the therapeutic applications a cultivation and proliferation of the cells is useful.

Preferably, the following step is carried out before the loading step:

- Cultivation of the immunocompetent cells extending over the cultivation period until a predetermined number of immunocompetent cells per unit volume is reached.

It is also advantageous if the following step is carried out after the loading step of the immunocompetent cells:

- Cultivation of the immunocompetent cells loaded with nanoparticles and/or the cytotoxic substance over a cultivation period until a predetermined number of immunocompetent cells is reached. E.g. a 1,000 to 100,000 times higher number of cells loaded with the nanoparticles is achieved if compared to the number of cells before the start of this cultivation step. The theranostic charge of the loaded immunocompetent cells is to be evenly distributed evenly to the daughter cells at each of the associated cell divisions.

Preferably, the growth of the loaded immunocompetent cells, as well as the distribution of the nanoparticles and/or the cytotoxic substance in the immunocompetent cells is controlled during the cultivation step, by, e.g., ionizing particle radiation emanating from the loaded immunocompetent cells being detected and visualized for this purpose. For stable isotopes this can be induced by thermal neutron radiation ($n_{th}$) or, in the case of the isotope $^{11}$B, by proton radiation. Radioactive payloads or tracers can be detected by radiation detectors.

Advantageously, before the step of providing the immunocompetent cells, a mutation is determined that is specific for the patient-specific genetically modified cells. This specific mutation is located in particular in a coding gene that codes for an antigen that is presented on the outside of these cells, for example the tumor cells. Furthermore, a gene that confers specificity for this mutation is provided for transfection of the immunocompetent cells and the immunocompetent cells are transfected with this gene so that the immunocompetent cells can specifically attack the patient-specific genetically modified cells of the patient.

According to a further aspect of the invention, the above-mentioned object is solved by immunocompetent cells, in particular T cells, in particular $\gamma\delta$ T cells, in particular, theranostic $\gamma\delta$ CAR-T cells, which are loaded with nanoparticles and/or a cytotoxic substance according to the method described herein. The specific process may include some or all of the features of the method as described above.

According to another aspect of the invention, the above object is further solved by immunocompetent cells having some or all of the features mentioned with respect to immunocompetent cells for use in a method for any theranostic therapy of patient-specific genetically modified cells, in particular viral genetically modified cells or a patient-specific malignant tumor. Preferably, the immunocompetent cells are for use in the treatment of cancer and/or malignant tumors.

According to an additional aspect of the invention, the above object is further solved by a medical composition comprising immunocompetent cells having some or all of the features mentioned above with respect to said immunocompetent cells. The medical composition further comprises a carrier, preferably a liquid carrier, in which these cells are present. The carrier liquid is intended to be administered to a patient in need thereof. The medical composition allows the immunocompetent cells to be infused to the patient in the carrier liquid which is specifically designed to be administered to a patient as it will be apparent to the skilled person. In particularly preferred embodiments, the medical composition is used in the treatment of cancer.

It is advantageous if the loaded immunocompetent cells are designed to distribute themselves within the patient's body within a maximum of 60 minutes, preferably within a maximum of 30 minutes, in such a way that their distribution in the patient's body can be detected with spatial resolution.

In addition, it is advantageous if the loaded immunocompetent cells can be detected in a spatially resolved manner in that they have a visualizable marker and/or ionizing particle radiation emanating from them can be detected, in the case of stable isotopes (e.g. $^{10}$B) after prior exposure to thermal neutron radiation ($n_{th}$) or, in the case of $^{11}$B, to proton radiation. Therefore, in preferred embodiments, in the case of $^{10}$B an exposure to thermal neutron radiation is applied and in the case of $^{11}$B a proton beam is applied.

A further aspect of the invention relates to a method for the preparation of genetically transfected immunocompetent cells loaded with nanoparticles and/or a cytotoxic substance for the theranostic treatment of patient-specific genetically modified cells, in particular of virally genetically modified cells or of a patient-specific genetically modified cell, malignant tumors in an affected patient, comprising the following steps:

a. Providing immunocompetent cells that have been transfected with at least one gene whose gene product confers specificity for the immunocompetent cells with respect to the patient-specific genetically modified cells;

b. loading the immunocompetent cells with the nanoparticles and/or the cytotoxic agent, comprising the steps of:

i. Applying the nanoparticles and/or the cytotoxic substance to the immunocompetent cells or mixing the nanoparticles and/or the cytotoxic substance with the immunocompetent cells in a carrier liquid to form a cell solution;

ii. Poration, in particular by sonoporation, electroporation or laserporation, of the immunocompetent cells in order to increase the permeability of the cell membranes of the immunocompetent cells for the nanoparticles and/or the

cytotoxic substance and thereby loading the immunocompetent cells with the nanoparticles and/or the cytotoxic substance.

Further details and preferred embodiments of this aspect of the invention correspond to the details and preferred embodiments as described above.

In particularly preferred embodiments, the nanoparticles and/or the cytotoxic substance is at least one stable isotope and/or at least one radioisotope, wherein preferably the at least one stable isotope and/or the at least one radioisotope is selected from the following group of stable isotopes or radioisotopes: $^{10}$B, $^{11}$B, $^{nat}$B, $^{157}$Gd, $^{99m}$Tc, $^{177}$Lu, $^{18}$F and $^{211}$At or mixtures thereof. In a particularly preferred manner, $^{10}$B and/or $^{157}$Gd is used for loading the immunocompetent cells.

In preferred embodiments of this aspect of the invention the immunocompetent cells were transfected in addition to at least one gene for the specificity of the immunocompetent cells towards the patient-specific genetically modified cells with a set of genes which is responsible for at least part of a chimeric antigen Receptor (CAR). Therein, the CAR comprises at least one extracellular specificity-imparting scFv antibody domain, an intracellular T cell activation domain, and a transmembrane domain connecting the scFv antibody domain and the T cell activation domain and anchoring the protein in the cell membrane, wherein the specificity-conferring gene encodes the scFv antibody domain.

Preferably, the immunocompetent cells are T cells, in particular $\gamma\delta$ T cells, in particular theranostic $\gamma\delta$ CAR-T cells.

The loading step b. is preferably carried out by sonoporation and, preferably, sonporation is performed in the presence of microbubbles. In preferred embodiments with respect to the microbubbles the following features are present:

a. There are free microbubbles in the cell solution and/or

b. the nanoparticles or the cytotoxic substance are coupled to the microbubbles prior to the sonoporation step, wherein the nanoparticles or the cytotoxic substance are coupled to the microbubbles in such a way that, after coupling, the nanoparticles or the cytotoxic substance are located in an interior space of the microbubbles, embedded in a microbubble membrane or covalently bound to the microbubble membrane from the outside.

Preferably, the step of coupling the nanoparticles and/or the cytotoxic substance with the microbubbles is carried out before or after the application or mixing of the nanoparticles or the cytotoxic substance with the immunocompetent cells.

In especially preferred embodiment of this aspect of the invention the charge of the loaded immunocompetent cells per unit volume is determined by the nanoparticles and/or the cytotoxic substance and the load is distributed evenly to the daughter cells at each cell division of the cells during culturing and proliferation of the cells.

In an especially preferred embodiment of this aspect of the invention, prior to the step of providing the immunocompetent cells, a method for preparing the immunocompetent cells for the patient is carried out as already outlined above. This encompasses the use of a specific mutation in a coding gene of an antigen presented on the cell membrane of these cells. The latter is determined, and a gene mediating specificity for this mutation is provided for transfection of the immunocompetent cells, and the immunocompetent cells are transfected with this gene.

According to a further aspect of the invention, the above-mentioned object is solved by immunocompetent cells which are loaded with nanoparticles and/or a cytotoxic substance and which can be produced according to said method described above. The immunocompetent cells are in particular T cells, preferably $\gamma\delta$ T cells. CAR-T cells are particularly preferred, especially theranostic $\gamma\delta$ CAR-T cells.

The invention further relates to a medical composition which comprises said loaded and immunocompetent cells. Said loaded and immunocompetent cells are obtained by said method for the preparation of genetically transfected immunocompetent cells loaded with nanoparticles and/or a cytotoxic substance as described above.

In preferred embodiments of said medical composition the loaded immunocompetent cells can be detected with temporal and spatial resolution in that they have a visualizable marker and/or ionizing particle radiation emanating from them that can be detected. In the case of stable isotopes like $^{10}B$ after prior exposure to thermal/epithermal neutron radiation ($n_{th}$) or in the case of $^{11}B$ to proton radiation.

According to a further aspect of the invention, the above object is solved by immunocompetent cells having some or all of the features mentioned with respect to the immunocompetent cells for use in a method for the theranostic treatment of patient-specific genetically modified cells, in particular viral genetically modified cells or a patient-specific malignant tumor, in an affected patient. Preferably, the immunocompetent cells loaded according to the invention are intended for the treatment of cancer diseases.

Further features and advantages of the invention result from the following description of further embodiments in connection with the drawings. The individual features here can each be implemented individually or in combination with each other.

BRIEF DESCRIPTION OF THE DRAWINGS

In the figures it is shown:

Figure 1          a schematic representation of a $\gamma\delta$ CAR-T cell;

Figure 2          a schematic representation of nanoparticle-carrying microbubbles;

Figures 3a) to d)   schematic and theoretical representation of a charging process using sonoporation;

Figure 4          representation of embodiments of $\gamma\delta$ CAR-T cells, which are hereinafter referred to as "T-ninja cells";

Figure 5          a schematic representation of a sonoporation charging device;

Figure 6          a schematic representation of a uniform distribution of nanoparticles and/or a cytotoxic substance to daughter cells after division of the T-ninja cells;

Figure 7          a schematic representation of an activity control of loaded T-ninja cells;

Figure 8          a schematic representation of the *modus operandi* of a loaded T-ninja cell *in vivo;*

Figure 9          a schematic representation of an interaction between a loaded T-ninja cell and a cancer cell;

Figure 10a) and b)   a schematic representation of a cancer cell after treatment with the loaded T-ninja cells.

DESCRIPTION OF EXEMPLARY EMBODIMENTS

In the following, embodiments of the invention are explained in more detail with reference to the drawings.

The figures show embodiments of the invention, and the following explanations relate to these embodiments. It is clear to a skilled expert that the explanations of the specific embodiments of the invention are in no way restrictive of the general idea of the invention.

Specifically shown in the figures is a functional design based on genetically modified immunocompetent cells as allogeneic $\gamma\delta$ CAR-T cells, but autologous applications are not excluded where necessary.

An effective expansion and distribution method for allogeneic transfer is known, for example, from WO 2016005752 A1, and has led to positive results in clinical studies. The procedure described there begins with the initial collection of $\gamma\delta$ T cells taken from a healthy first donor or, for example, umbilical cord blood. An enrichment to 99% of $\gamma\delta$ T cells is then achieved before multiplication by several orders of magnitude in this process.

Quantities of $2.5\times10^{10}$ cells can be obtained from a single donor within 10 to 18 days. The optimum time for a loading with nanoparticles and/or a cytotoxic substance is agreed upon by clinicians after thorough testing with the clinics in which the expansion and enrichment process of the $\gamma\delta$ CAR-T cells is carried out.

An international network of dedicated allogeneic supply chains in Europe, the US, and Asia, which includes cryogenic facilities, will enable the storage of $\gamma\delta$ CAR-T cells, which can be used for therapies for up to two to three weeks after exponential multiplication.

The loading of the nanoparticles and/or a cytotoxic substance, according to the invention, represents an advantageous extension of the variety of emerging $\gamma\delta$ CAR-T cell therapies. For example, the present embodiments utilize the unique cancer recognition capability of $\gamma\delta$-CAR-T cells, which can be tuned to target only specific cancer cells. With the support cytokines (for example, IL-2 or IL-12), the cancer cells are targeted so that the side effects caused by an attack on the patient's healthy cells are greatly reduced using the cells according to the invention and the manufacturing process described.

Patient-specific genetically modified cells in the form of malignant tumor cells in the embodiments depicted are initially attacked by T-cell cytotoxic substances. They can then be further attacked by releasing the loaded nanoparticles or the loaded cytotoxic substance. The nanoparticles may, for example, be nanoparticles that can be activated later by supportive radiotherapy.

Nanoparticle technologies have a significant impact on the development of therapeutic and diagnostic agents (theranostics). At the interface between treatment and diagnosis, clinically effective formulations have been developed. Most of these new developments see nanoparticles as carriers of drugs or contrast agents. Various contrast agents have been developed in clinical examinations to observe their interactions with the specific disease. Most commonly, optically active small molecules, metals and metal oxides, ultrasound contrast agents, and radionuclides are used. Theranostics is of great importance for active substances that act on molecular biomarkers of disease and will, therefore, contribute to personalized medicine for efficient treatments.

The focus for these nanoparticles is on specific isotopes relevant to cancer treatment, with $^{10}$B, which is used in boron neutron capture therapy (BNCT) (Malouff et al. (Feb. 2021): "Boron Neutron Capture Therapy: A Review of Clinical Applications"). The most important feature of the nanoparticles described is that they can simultaneously loaded with other nanoparticles and/or a cytotoxic substance but also can be loaded separately or together with radiomarkers. This enables the monitoring and controlling of the density and distribution of the $\gamma\delta$ CAR-T cell loading. Related times can be measured and optimized, e.g. the total time needed for the treatment to be most effective. This can also applied before any irradiation, for example, the aforementioned neutron radiation.

The nanoparticles can be any nanoparticles that are currently relevant in nuclear medicine. At present, as already mentioned, $^{10}$B is primarily used for BNCT. Gadolinium $^{157}$Gd is very interesting for a similar procedure; the so-called GdNCT in reference to BNCT, as this isotope has the highest neutron capture cross-section in nature; a much lower level of irradiation is required as part of a GdNCT. Therefore, even very sensitive regions of the body can be treated. Both isotopes are not radioactive by nature but are only excited to decay when they are activated by an external neutron source, for example, by an accelerator or a nuclear reactor. The maximum effective cross-section for triggering the decay is in the epithermal to thermal energy range of the neutrons, corresponding to a kinetic energy of about 0.025 eV.

The BNCT utilizes the fission reaction that occurs when non-radioactive $^{10}$B is irradiated with thermal or epithermal neutrons $n_{th}$. The tissue of the body is transparent to such neutrons so that they penetrate the tissue unhindered.

However, the epithermal neutrons lose a small amount of energy when they penetrate tissue, so they become thermal neutrons - they are "thermalized".

$$^{10}\text{B} + n_{th} \rightarrow {}^{4}\text{He} + {}^{7}\text{Li} + 2.8 \text{ MeV} \qquad (1)$$

According to **Formula 1,** a high-energy $\alpha$-particle and a $^{7}$Li-ion are generated, both of which lose kinetic energy determined by the Q value (2.8 MeV) of the reaction through linear energy transfer (LET) as they pass through the cell. The $^{7}$Li and the lighter $^{4}$He-ions penetrate for a maximum range of ~ 4 $\mu$m for $^{7}$Li and ~ 9 $\mu$m for $^{4}$He inside (a single) cell. Since these lengths correspond to the diameter of a human cell, the lethality of the capture reaction is only limited to the cell itself, and/or cells in the immediate vicinity of the boron nanoparticles. The success of BNCT, therefore, depends on the selective delivery of sufficiently high quantities of $^{10}$B to the tumor cells themselves, whereby only small quantities are localized in the surrounding healthy tissue. This is one of the core objectives of the treatment with the genetically modified and loaded immunocompetent cells of the present invention.

As of today, in the best case, the boron load of a patient-specific genetically modified cell, in particular a cancer cell, can be increased by only three to four times compared to the neighboring healthy surrounding cells. According to the invention, this ratio can be enhanced by four to six orders of magnitude while at the same time reducing side effects with the presented method. The limitation of the previous methods is disadvantageous because normal tissue tolerates the flow of slow neutrons due to the relatively low nuclear capture reactions that occur with hydrogen and nitrogen. In comparison, the undesirable radiation doses associated with thermal and epithermal neutron irradiation, to which healthy tissue is exposed, are minimized by the methods of the invention. For BNCT to be effective, only a single fission of a $^{10}B$ isotope is sufficient to disrupt a patient-specific genetically modified cell, in particular a tumor-like malignant cell.

The isotope $^{10}B$ isotope makes up around 20% of the low-priced natural boron ($^{nat}B$) thus, nanoparticles carry also a large proportion of $^{11}B$, which is also stable and can be used for radiotherapy. $^{11}B$ nanoparticles have a large effective radius for proton radiation with an energy of approx. 600 keV.

This, in turn, means that, i.e., immunocompetent cells loaded with them in the form of $\gamma\delta$ CAR-T cells can also be used for proton radiation therapy, although most of the benefits for BNCT will be lost.

In addition to the non-radioactive isotopes mentioned above, other radioisotopes and/or isomers can be considered for cancer theranostics, for example, e.g., $^{99m}Tc$, $^{177}Lu$, $^{18}F$, and $^{211}At$. All other isotopes and/or isomers of current medical relevance for cancer or other treatments that target patient-specific genetically modified cells that have a potentially pathogenic potential are included in the proposed method. In general, these are either commonly used radiological isotopes or those currently being used in advanced stages of clinical trials, where they promise to treat various malignant diseases.

For the isotopes described in the previous paragraph, the radioactive activity differs as a function of time A(t), which is referred to here for the described biological activity of the $\gamma\delta$ CAR-T cells.

The activity A(t) describes the decay of a radioactive sample per second and is defined by

$$A(t) = \lambda \times N(t) \qquad\qquad (2)$$

where $N(t) = N_0 \times \exp(-\lambda \times t)$ is the number of isotopes in the sample, $\lambda$ is the decay constant for a particular isotope, and $N_0$ is the number of isotopes at an arbitrarily chosen reference time $t_0$.

Clinicians can determine the time frame and the number of nanoparticles of the above-mentioned isotopes with comparatively high precision. In contrast to A(t), biological activation does not follow such a simple exponential rule. Instead, it depends on various controllable external factors, such as cell growth, survival rate, and cryopreservation for later "revival".

The dose rates required to activate a therapeutic amount of radioactive decay are calculated based on continuous or pulsed sources currently available at specially enhanced nuclear reactor sites, radio frequency (RF) accelerators, or tandem accelerators. Furthermore, there will soon be modern laser-driven accelerators available for clinical use, delivering comparatively high temporal beam doses and thus minimizing the total time for patient irradiation.

The activities A(t) of the theranostic sample are adjusted accordingly for each treatment to determine the optimal dose rate:

$$D = dE/dm = (1/\rho)\ dE/dV \qquad\qquad (3)$$

where dE describes the energy released within a mass segment $dm$ in the patient's body with a specific density of $\rho$, which extends over a volume element of $dV$.

For precise calculations, the heterogeneous structures of the tissue would have to be divided into a number of inhomogenity groups $D_i$, before they are summed up to D to form a treatment plan advised by clinicians. The dose rate D is expressed in microgray per hour [$\mu$Gy/h] and converted into the equivalent dose rate HT, given in Sieverts per hour [Sv/h] for one treatment..

**Process steps in detail**

Step 1 Loading of genetically modified immunocompetent cells in the form of $\gamma\delta$ CAR-T cells with nanoparticles and/or a cytotoxic substance by sonoporation

In the following, reference is made to the figures and the reference symbols shown therein, whereby the figures only show specific embodiments of the invention are described below. Reference signs are generally only assigned once in each the following. However, it will be readily apparent that the same reference signs identify the same structures in the figures, unless another reference sign has been explicitly assigned.

Genetically modified immunocompetent cells in the form of cultured $\gamma\delta$ CAR-T cells (1), e.g. v$\gamma$9v$\delta$2 T cells, which have a co-stimulatory chimeric antigen receptor (CAR) (2) inserted into their cell membrane by genetic transfection

and a native $\gamma\delta$-T-cell receptor ($\gamma\delta$-TCR) (3) can recognize the endogenous isopentenyl pyrophosphate (IPP) antigens on the cell surface of cancer cells. They are removed at an early stage of their expansion process in suitable batch sizes and placed on a Petri dish.

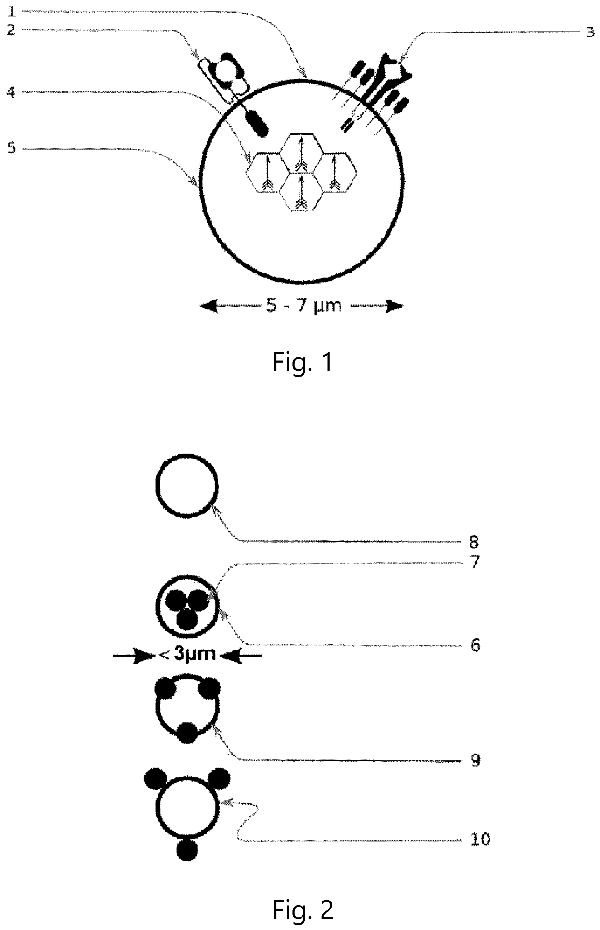

Their cell membrane (5) contains cytotoxins (4) (see **Figure 1**). A typical set of cells at an early stage of the expansion process is in the range of $10^6$ to $10^8$. The dish contains an Opticell™ sample holder or a similar arrangement using a Petri dish.

The nanoparticles and/or the cytotoxic substance can be added as a layer of free atoms or molecules, whereby they can also, as can be seen in **Figure 2,** be previously integrated into so-called microbubbles. If they are added as a layer of free nanoparticles or molecules is present, a defined layer of empty microbubbles can then be added. The cells are located in a carrier liquid, and a cell solution is formed by adding the nanoparticles and/or the cytotoxic substance.

The nanoparticles or the cytotoxic substance can, therefore, be freely present. They can also be present in the interior of the microbubbles (6), preferably chemically bound to each other or, for example, in the form of a chelate complex (7), in microbubble membranes, in particular mechanically embedded (9) or covalently bound to the microbubbles (10) from the outside. A preferred diameter of a microbubble is approx. 2 $\mu$m (preferably less than 3 $\mu$m). Preferably, the nanoparticles and/or the cytotoxic substance and the immunocompetent cells are added to the Petri dish in layers. It is also advantageous to keep a diameter tolerance of the microbubbles low by appropriate pre-filtration, i.e. that all microbubbles have essentially the same size.

In the present embodiment, as soon as the load samples are distributed, the Petri dish will get reversed, so that the $\gamma\delta$ CAR-T cells are placed at the top of the arrangement.

The superimposed layers of this preparation are then compacted using ultrasonic pressure so that the distances between the components of the layers are brought to a predetermined and comparatively small average distance.

The pressure P(t) applied by the ultrasonic transducer drives the nanoparticles, the cytotoxic substance and/or the loaded microbubbles into the vicinity of the $\gamma\delta$ CAR-T cells up to an optimum distance from the $\gamma\delta$ CAR-T cells so that the microbubbles can release their charge to the $\gamma\delta$ CAR-T cells.

P(t) and the time duration $t_{total}$ of the effect of the ultrasonic pressure on the cell solution are partly based on empirical data derived from laboratory work, considering radioactive and biological activation. The exact process settings are specific to the microbubbles and the specialized $\gamma\delta$ CAR-T cells to be used in treatment.

The implosion of a microbubble, which is the process for delivering the payload, is determined by the Rayleigh-Plesset formula, which determines the change in the bubble radius R(t) over time:

$$R(t)\frac{d^2 R(t)}{dt^2} + \frac{3}{2}\left(\frac{dR(t)}{dt}\right)^2 + \frac{4v_L}{R(t)}\frac{dR(t)}{dt} + \frac{2\gamma}{\rho_L R(t)} + \frac{\Delta P(t)}{\rho_L} = 0 \qquad (4)$$

where $\rho_L$ is the density of the surrounding liquid, $v_L$ is the kinematic viscosity of the surrounding liquid, $\gamma$ is the surface tension of the microbubble-liquid interface, $\Delta P(t) = P_\infty(t) - P_B(t)$ is the pressure difference between the uniform pressure inside the bubble $P_B(t)$ and $P_\infty(t)$ is the external pressure "infinitely" far from the bubble. Formula 4 makes it possible to optimize the transducer's trigger signal in relation to the time of disintegration of a microbubble, whereby the free nanoparticles or the nanoparticles bound in microbubbles or the cytotoxic substance enter the T cells.

In order to be able to carry out at least partial permeabilization of the cell membranes of the $\gamma\delta$ CAR-T cells used in the present example in a gentle and controlled manner, it is advantageous in the present embodiment if the following steps are carried out.

All components involved in the sonoporation process can be subjected to precise tolerance control of the dimensions, as these have a significant influence on the intrinsic modes of the setup. In particular, this requirement concerns the diameter of the microbubbles used, so that they are brought to a $\pm$ 10% tolerance by a micropore filter film before use.

Any expert understands that the device settings are precisely maintained and documented and that electrical or electromagnetic fields are preferably used to control the reclosure of the cell membranes; for example, in a closed cell membrane, a feedback loop can be used. Since $P(t)$ in Formula 4 is periodic in nature: P(t') = $P$(t + $t_{period}$), and even if a sinusoidal ultrasonic waveform provides good results, an optimized pulse profile shape with constant period may be more favorable for optimal sonoporation in terms of the set goals. Standard microbubbles produced industrially and available on the market are assumed.

$P$(t) is optimized with respect to the amplitude $P_0$(t), the period $t_{period}$ and the total duration of the sonoporation process ($t_{total}$). For each specific variety of $\gamma\delta$ CAR-T cells, cellular responses such as cell membrane permeability and cytoskeletal fragmentation are to be investigated with respect to the dependence on nuclear parameters such as acoustic driving pressure $P$(t) and the distances between the emitting microbubbles.

For a given bubble diameter of ~ 2 $\mu$m and a cell with dimensions of a few $\mu$m, a two-dimensional simulation model according to Formula 4 leads to optimal values of $P_0$(t) ~ 400 hPa. A typical time for the endocytosis of the contents of the microbubbles into the immunocompetent cells is less than 1 $\mu$s. One aim of the simulations and the design is to make the carefully prepared $\gamma\delta$ CAR-T cells from unnecessary stress and thus optimize the effectiveness of the manufacturing process.

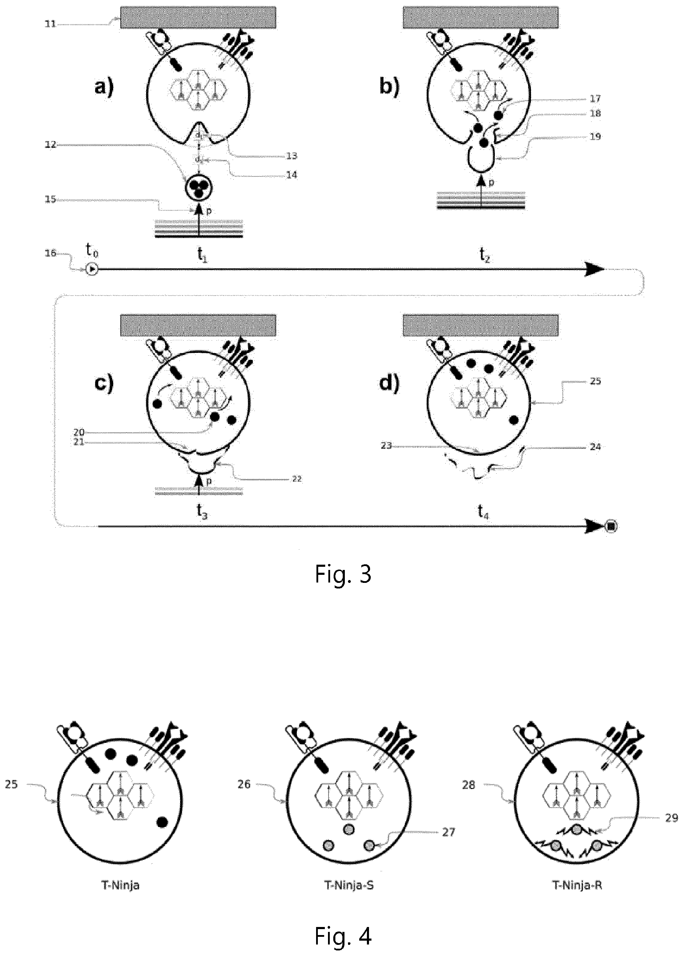

The sonoporation process, as can be seen in **Figure 3,** is divided into four phases $t_{1,2,3,4}$ after the ultrasonic transducer is switched on. After a sample holder, in this case, an Opticell™ sample holder (11), has been filled with a sufficient number of $\gamma\delta$ CAR T cells are used as genetically modified immunocompetent cells, the arrangement is reversed so that the free nanoparticles and/or the cytotoxic substance can diffuse from the loaded microbubbles (12) in the direction of the $\gamma\delta$ CAR-T cells.

In the immediate vicinity of the microbubbles, the application of ultrasound pressure via the ultrasound transducers leads to an indentation (13) with the depth dt of the cell membranes of the $\gamma\delta$ CAR-T cells. The size of it depends on the distance ds between the microbubbles and the $\gamma\delta$ CAR-T cells; see the reference sign (14). Both parameters dt and $d_s$ are functions of the applied pressure $P(t)$, see the reference sign 15, and thus implicitly of the time t, see the reference sign (16).

The nanoparticles and/or the cytotoxic substance, see the reference sign (17) in **Figure 3b,** penetrate into the $\gamma\delta$ CAR-T cell as soon as the sonoporation has led to an increase in the permeability of the cell membranes of the $\gamma\delta$ CAR-T cells, see the reference sign (18). The microbubbles (12), see reference sign (19), then disintegrate at time $t_2$, typically in less than 1.0 $\mu$s after the start of exposure to $P(t)$. Parallel to the disintegration of the microbubbles, the charge carried by the microbubbles (12) is transferred to the immunocompetent cells in the form of the $\gamma\delta$ CAR-T cells, for example by endocytosis, free diffusion and/or osmotic pressure into the $\gamma\delta$ CAR-T cells.

This completes the loading process for the $\gamma\delta$ CAR-T cells. After the loading of the $\gamma\delta$ CAR-T cells, the nanoparticles and/or the cytotoxic substance are located inside the $\gamma\delta$ CAR-T cells, see the reference sign (20) in **Figure 3c.** Thereafter, the cell membranes of the $\gamma\delta$ CAR-T cells close again, see reference line (21). Then the pressure has been reduced at time $t_3$. The decay of the microbubbles at this point has progressed to such an extent that the microbubbles have disintegrated into smaller and no longer usable microbubbles, see the reference sign (22).

**Figure 3d** illustrates the state of the $\gamma\delta$ CAR-T cells at the end of the loading process at time $t_4$, at which the cell membranes have closed again, see reference sign (23), and the microbubbles have completely disintegrated, which is referred to by the reference sign 24.

Residual microbubbles are filtered using standard molecular biological methods. The loading depends on the radiological and biological activity of the patient-specific genetically modified cells in the patient, which is determined in advance for the treatment. It is assumed that the loading process takes place at an earlier stage of cell culture growth. Therefore, the intended payload per cell should not exceed the biologically acceptable, i.e. non-lethal, threshold. For boron loading, this would correspond to a payload of approximately 0.13 picograms [pg] per cell. This corresponds to $3 \times 10^{10}$ atoms of natural boron, per cell.

For radioisotopes, the dose exposure for the treating person should be taken into account. Patient-specific exposure is also determined by factors such as the proliferation of $\gamma\delta$ CAR-T cells in a patient's body and a period of two to four days during which they usually accumulate around the patient-specific genetically modified cells, such as tumor cells.

For a more straightforward description, the $\gamma\delta$ CAR-T cells loaded with the nanoparticles and/or the cytotoxic substance and used here are referred to as "T-ninjas". As illustrated in **Figure 4,** for a more precise differentiation, the T-ninjas are also divided into three subgroups according to the nature of the loaded nanoparticles and/or the loaded cytotoxic substance.

a) T-ninja-R or $\gamma\delta$ CAR-T-R, see the reference sign (28), if the T-ninjas are loaded with a cytotoxic substance, in the present case radioisotopes (29) such as $^{99m}$Tc or $^{211}$At. By these radioisotopes an immediate radiologically therapy is of relevance for the first stage of a combined therapy against patient-specific altered cells. This comprises a cancer therapy without an activator beam, or, for example, a neutron beam or a proton beam.

b) T-ninja-S or $\gamma\delta$ CAR-T-S, see the reference sign (26), if the T-ninjas are loaded with nanoparticles in the form of stable isotopes (27), such as $^{10}$B or $^{157}$Gd. For that cases, the radiologically relevant nuclear reaction against can be initiated at a desired point in time, for example in a combined therapy.

c) T-ninja-SR or $\gamma\delta$ CAR-T-SR, which are not explicitly illustrated, as a combination of the T-ninja-R and T-ninja-S cells, whereby the combination of the cytotoxic substance present in the form of radioisotopes and nanoparticles present in the form of stable isotopes can be used particularly efficiently and flexibly.

In addition, all the T-ninjas described can also be used with additional substances at the same time, which will enable better monitoring and control of the manufacturing process and also the treatment of patients in certain phases of therapy.

Ultrasonic pulses with a total duration of 20 s to 40 s are only applied in several cycles, for example, in order to increase the achievable payload of the $\gamma\delta$ CAR-T cells under constant observation and to determine the actual own modes of the set-up. The microbubbles themselves are located in a phosphate buffer solution, for example. A total quantity of six million microbubbles per ml is considered the ideal concentration for the sonoporation process.

After a total number of 100,000 ultrasonic periods, a loading efficiency of the microbubbles of at least 10% can be assumed if the sound pressure profile, the duration of the sound pressure periods, and the transducer geometry have been optimized.

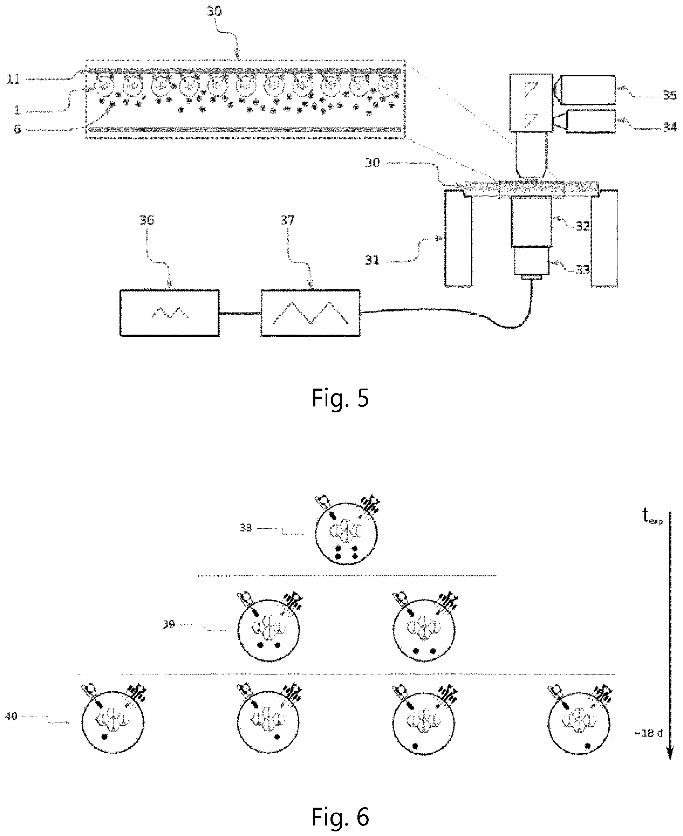

As can be seen from **Figure 5,** a preferred device for loading the $\gamma\delta$ CAR-T cells is a sonoporation charging device, with which preferably a simultaneous image monitoring can be applied that can observe the loading of the nanoparticles and/or the cytotoxic substance. This loading apparatus assembly (LAA), also referred to as such by specialists, comprises a closed Petri dish (30), fastening elements (31), a coupling gel (32), and an ultrasonic transducer, which is not specifically described.

The latter provides a power of >200 W at frequencies of up to several MHz, see the reference sign (33), with a focal spot of > 18 mm. The ultrasonic transducer is driven from a waveform generator (36), which is amplified by a class A broadband amplifier (37) to provide a linear output in the frequency range from 10 kHz to 12 MHz for any waveform.

A typical setting comprises a curve with a constant period of P(t) with an average value of < $P\infty$(t) and > =400 kPa at a frequency of > 1 MHz. The sonoporation process can be monitored using a microscope setup with a monochromatic light source (34) and a CCD camera (35).

An expert will be aware that the loading of the immunocompetent cells essentially depends on increasing the permeability of the cell membranes so that the nanoparticles that may be coupled to the microbubbles or the cytotoxic substance coupled to the microbubbles can enter the cell lumen of the immunocompetent cells. Therefore, instead of sonoporation, electroporation or laser poration may be used, whereby sonoporation is comparatively advantageous, as explained in detail above.

Step 2 Distribution of nanoparticles and/or cytotoxic substance to daughter cells of loaded T-ninja cells after sonoporation

As can be seen in **Figure 6,** starting from the previously prepared T-ninja cell culture, which was created in the LAA under optimal growth conditions, the payload of each T-ninja cell in the cell culture is divided equally from the first generation ($n_g$ = 1) (38) to the subsequent generations (39), (40) during proliferation. With each mitotic cell division, the individual cell payload of the daughter cells is reduced by half compared to the previous generation. The proliferation of the cells can take place over a period of up to three weeks. Constant monitoring of this proliferation phase ensures that the proliferation phase is terminated as soon as stagnation of cell division is detected.

$$PL(n_g) = \left(\frac{1}{2}PL(1)\right)^{n_g} \qquad (5)$$

Clinical needs and ongoing monitoring will inform the optimal timing and extent of cell proliferation and determine the final average payload of engineered T-ninja cells based on the Formula 5. In the case of radioisotopes, it is advantageous if the loading of the T-ninja cells is carried out immediately before the infusion of the T-ninja cells into the patient's body, as the T-ninja cells do not survive for long due to the ionizing particle radiation of the radioisotopes.

Step 3 Measurement of the payload of nanoparticles and/or a cytotoxic substance in sonoporation- and microbubble-loaded T-ninja cells

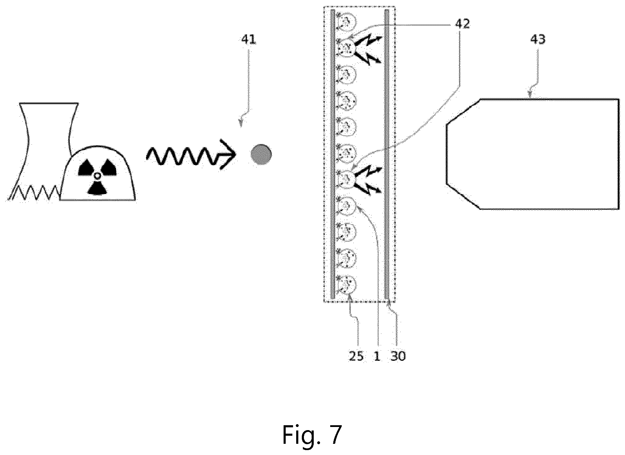

As can be seen from **Figure 7,** in the present embodiment example, monitoring and control of the payload, referred to below as the radiation dose D, is provided after each step of the T-ninja development.

In the present case, an activating electron, neutron, proton, or $\gamma$ beam (41) for a stable isotope such as $^{10}$B or $^{157}$Gd is used for this purpose, with which T-ninja-S cells (42) of the cell culture are loaded, but preferably a thermal or an epithermal neutron beam is used. Under the influence of the activation beam, a fission reaction according to Formula 1 is initiated in the T-ninja-S cells, which can be monitored with a detector (43). T-ninja-R cells, which are not illustrated here, and which are loaded with a cytotoxic substance in the form of a radioisotope such as $^{18}$F, do not require such an activation beam, as they already emit ionizing particle radiation. However, they can be detected but can be observed in exactly the same way with the same detectors. The detector (43) can be represented by a high-purity germanium detector (HPGe) or a lanthanum bromide detector (LaBr$_3$). However, detectors with better spatial and temporal resolution, which are currently under development, are preferred for use in this method.

In addition, suitable measurements of the density and distribution of the nanoparticles and/or the cytotoxic substance in the cell culture can be undertaken.

Step 4 Preparation, recognition, and administration of nanoparticles and/or a cytotoxic substance using γδ CAR-T cells loaded with those entities

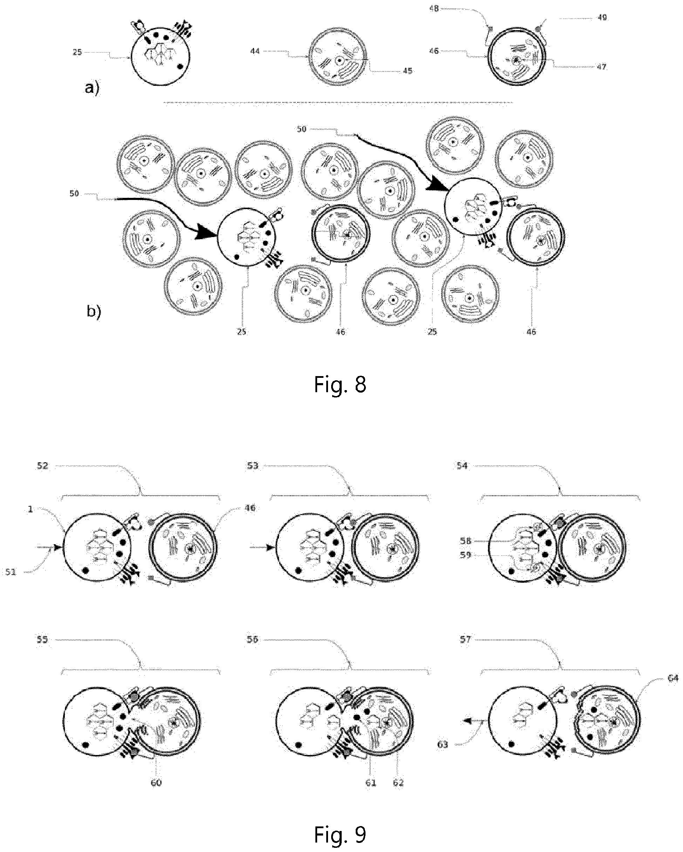

The core vehicles in a therapy against patient-specific genetically modified cells, for example a tumor or cells genetically modified by a viral infection, are, as already mentioned, summarized as T-ninja cells. In the following, reference will be made to **Figure 8.**

Due to the TCR and the CAR, T-ninja cells have an exceptionally high specificity for corresponding patient-specific genetically modified cells and "search" for the affected body cells (46). They attach themselves to these, see the reference sign (50), mediated by the specificity of the T-ninjas, for example, for a biological "stress signal" (48) and a target antigen (49).

The approach process of the T-ninja cells to the patient-specific modified cells can be divided into six sequences (52) to (57), displayed in **Figure 9.**

The sequence (52) shows the last stage of the recognition process of a cancer cell (46), whereby the reference sign (51) indicates the direction of the T-ninja movement. Sequence (53) shows an initial docking phase of the T-ninja cells, which is caused by the high specificity of the T-ninja cells with respect to the cancer cells. The sequence (54) shows an interaction of the CAR-T-cell receptor and the γδ T-cell receptor (γδ TCR) with the target antigens of the cancer cell as well as an excitation of two independent trigger signals for cytokine release of the T-ninja cells (58), (59).

The combination of the two intracellular signals activated by the CAR-T and the γδ T cell receptor "switch" the T-ninja cells into a cytokine-enhanced attack mode. These two signals induce a cellular response from the T-ninja cells, which is also known as the "kiss of death". This is illustrated in sequences (55) and (56).

Sequence (55) shows how the cell membrane of the cancer cell is opened by the T-ninja cell. The reference sign (56) refers to the actual "kiss-of-death", in which the T-ninja cells release their own, i.e. native, cytotoxic substances as well as charged nanoparticles and/or a cytotoxic substance as described above through osmotic pressure into the patient-specific genetically modified cells.

The reference sign (57) refers to a detachment process of the T-ninja cells. The T-ninja cells will then continue their path (63) in order to find further patient-specific genetically modified cells, such as cancer cells in the present case, of the same type. The unhealthy cell remains with the nanoparticles and/or the cytotoxic substance enclosed inside its body (64).

Step 5 Induction of destruction of the patient-specific genetically modified cells: Infusion of preparations and/or triggering by means of particle beams

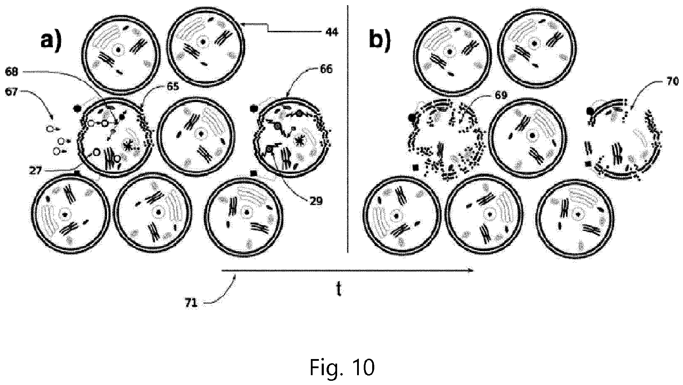

Reference is made below to **Figure 10.** A patient-specific genetically modified cell, i.e. an unhealthy cell 66 (UHC type B), is produced soon after the uptake of a cytotoxic substance in the form of radioisotopes (29) from T-ninja-R cells, even without a stimulus by the activation beam, see the reference sign (70) and **Figure 10a.** On the other hand, such a cell that has received nanoparticles in the form of stable isotopes (27) by a T-ninja-S cell requires the activation beam (67) to start the fission of the isotopes (68) (e.g. $^{10}$B), destroying the patient-specific genetically modified cell (69), see **Figure 10b.**

The first part of the radiological therapy is preferably carried out with radioisotopes, and the second part of the therapy begins only after the activation beam has been switched on at a time determined during the therapy; in any case after the first part of the therapy and depending on the success achieved by the latter. The nanoparticles can be used in separate T-ninja cultures, or together in a single one, so that T-ninja-SR cells are used instead of T-ninja-R and T-ninja-S cells.

Overview of an embodiment of the invention from the cultivation of immunocompetent cells to theranostic therapy against patient-specific genetically modified cells in the patient

An example of an embodiment of the immunocompetent cells according to the invention and an example of the application according to the invention in a theranostic therapy against patient-specific genetically modified cells in the affected patient following the production can be summarized as follows:

a) Due to the selectivity of the procedure, a histologic and/or a histopathological examination of the patient's affected tissue is carried out, for example, to determine which specific cancer the patient has.

b) Preferably, allogeneic γδ T-cells in sufficient numbers and quality are used according to the results of a), i.e. - to stay with the example - specifically for the respective cancer of the patient, transfected with a CAR gene that, among other things, codes for an scFv antibody domain that mediates specificity for respective cancer, whereby patient-specific γδ CAR-T cells are obtained.

c) The patient-specific $\gamma\delta$ CAR-T cells transfected in this way are cultivated and expanded. The products are then subjected to a testing phase while they are continuously monitored and controlled in order to select the right time for the next step in the process.

d) The nanoparticles and/or the cytotoxic substance are preferably applied in layers or mixed randomly with the $\gamma\delta$ CAR-T cells and mixed with a microbubble solution to obtain a cell solution in which the nanoparticles and/or the cytotoxic substance are coupled with the microbubbles.

e) Sonoporation of the cell solution with predetermined ultrasound parameters and under constant observation, with or without feedback control of the poration process. Alternatively, poration can also be achieved using laser poration. This results in a temporary permeabilization of the cell membranes of the $\gamma\delta$ CAR-T cells, which enables the uptake of the surrounding nanoparticles and/or the cytotoxic substance.

f) For better control of the partial permeabilization of the cell membranes of the $\gamma\delta$ CAR-T cells, sonoporation is carried out in the presence of stationary or non-stationary electric or electromagnetic fields, whereby both sonoporation and the field control described here can be carried out with or without feedback control.

g) The mixture thus prepared is subjected to a further cultivation phase in which the number of loaded $\gamma\delta$ CAR-T cells usually increases by a factor of 1,000 to 100,000, whereby the load of a $\gamma\delta$ CAR-T cells is distributed evenly after cell division to the resulting daughter cells.

h) Step g) is carried out under constant observation, whereby, for example, the betaemitters $^{18}$F, $^{99m}$Tc, or $^{177}$Lu can accompany the cytotoxic substance. This allows the density and distribution function of the loading process to be observed in the cell solution. In the case for boron-loading the process observed by activation via the means of an thermal/epithermal neutron beam.

i) The prepared cell solution is mixed with a preferably isotonic infusion solution and administered as an infusion to the intended patient.

j) Usually, the infusion solution is distributed in the body within approx. 30 min, so that after a relatively short time after the infusion, the density and distribution of the loaded $\gamma\delta$ CAR-T cells in the body can be spatially determined with the aid of the loaded nanoparticles and/or the cytotoxic substance, so that, for example, the attacked target areas can be identified at regular intervals or continuously or can be monitored.

k) It should be noted that the $\gamma\delta$ CAR-T cells, irrespective of their load, deliver native cytotoxic substances to the affected cells after contact with the patient-specific genetically modified cells.

l) A slow, adapted approach is recommended to prevent the destroyed cells from overloading the excretory pathways, for example, due to kidney failure. The body uses the time until the next step of the therapy to remove the $\gamma\delta$ CAR-T cells that have not been absorbed but are still present in the infusion and/or the cytotoxic substance in a natural way.

m) Based on regular monitoring, the right time for the first Boron-Neutron-Capture-Therapy (BNCT) can be determined, and one or several therapies can be carried out at specific intervals in such a way that, depending on the localization and type of the patient-specific genetically modified cells, in particular the tumor, the BNCT is performed on a spatially limited area of the patient's body or possibly, as preferably in the case of hematological cancers, on the entire patient's body, whereby the BNCT repetition is also justified by any metastasizing tumor cells in the body after the first treatment. The neutron energy is selected so low that the affected tissue is reached by thermal neutrons with energies of 0.025 eV, for example, which minimizes the risk of radiation damage to the tissue.

n) During the execution of the BNCT, the resulting fission products can be used to localize and monitor the BNCT in real time.

o) The need for any repetitions of the procedure with or without changed parameters will be determined by the attending physician or clinic, whereby continuous monitoring ensures continuous success control.

**Claims**

1. Method for loading immunocompetent cells with nanoparticles and/or a cytotoxic substance for use in the theranostic treatment of patient-specific genetically modified cells, comprising the following steps:

   a. Providing immunocompetent cells;
   b. loading the immunocompetent cells with the nanoparticles and/or the cytotoxic substance, wherein the nanoparticles and/or the cytotoxic substance are nuclides for radiotherapy, comprising:

   i. applying the nanoparticles and/or the cytotoxic substance to the immunocompetent cells or mixing the nanoparticles and/or the cytotoxic substance with the immunocompetent cells in a liquid carrier;
   ii. Poration of the immunocompetent cells in order to increase the permeability of the cell membranes of the immunocompetent cells for the nanoparticles and/or the cytotoxic substance and thereby loading the immunocompetent cells with the nanoparticles and/or the cytotoxic substance.

2. Method according to claim 1, **characterized in that** the nanoparticles and/or the cytotoxic substance are at least one stable isotope and/or at least one radioisotope from the following group of stable isotopes or radioisotopes: $^{10}$B, $^{11}$B, $^{nat}$B, $^{157}$Gd, $^{99m}$Tc, $^{177}$Lu, $^{18}$F, and $^{211}$At, preferably $^{10}$B and/or $^{157}$Gd.

3. Method according to claim 1 or claim 2, **characterized in that** the poration is carried out by sonoporation or electroporation or laser poration, preferably by sonoporation.

4. Method according to one of the preceding claims, **characterized in that** the loading of the immunocompetent cells with nanoparticles and/or a cytotoxic substance is performed in the presence of microbubbles, wherein preferably the diameter of the microbubbles is 0.5 $\mu$m to 4 $\mu$m, preferably 1 $\mu$m to 3 $\mu$m, more preferably about 2 $\mu$m.

5. Method according to one of the preceding claims, **characterized in that** the immunocompetent cells are T cells.

6. Method according to one of the preceding claims, **characterized in that** the immunocompetent cells are genetically transfected cells that have been transfected with at least one gene whose gene product confers specificity for the immunocompetent cells with respect to the patient-specific genetically modified cells.

7. Method according to one of the preceding claims, **characterized in that** the immunocompetent cells are T cells, preferably CAR-T cells, more preferably $\gamma\delta$ CAR-T cells.

8. Method, according to one of the preceding claims, **characterized in** the poration is controlled by applying a stationary or a non-stationary electric or electromagnetic field during sonoporation, in particular, to control permeabilization of the membranes of the immunocompetent cells.

9. Method according to one of the preceding claims, **characterized in that** the following step is carried out before the loading step:

   a. Cultivation of the immunocompetent cells over a cultivation period until a predetermined number of immunocompetent cells is reached per unit volume.

10. Method according to one of the preceding claims, **characterized in that** the following step is carried out after the loading step of the immunocompetent cells:

    a. Cultivation of the immunocompetent cells loaded with the nanoparticles and/or the cytotoxic substance over a cultivation period until a predetermined number of immunocompetent cells is reached per unit volume, wherein preferably the predetermined number of immunocompetent cells is a multiplication by a factor of 1,000 to 100,000 compared to the number of immunocompetent cells before the start of this cultivation step.

11. Method according to claim 10 with reference back to claim 2, **characterized in that** the growth of the loaded immunocompetent cells as well as a distribution of the nanoparticles and/or the cytotoxic substance in the immunocompetent cells is monitored during the cultivation, wherein preferably an ionizing particle radiation emanating from the loaded immunocompetent cells is detected and preferably visualized.

12. Immunocompetent cells which are loaded with nanoparticles and/or a cytotoxic substance, obtainable by a method according to one of the claims 1 to 11.

13. Immunocompetent cells according to claim 12 for use in a method for the theranostic treatment of patient-specific genetically modified cells in an affected patient, preferably for use in the treatment of cancer and/or malignant tumors.

14. A medical composition comprising immunocompetent cells according to claim 12 and a carrier liquid, wherein the carrier liquid is intended to be administered to a patient.

15. The medical composition according to claim 14, **characterized in that** the loaded immunocompetent cells are designed to undergo distribution within the patient's body within a maximum of 60 minutes, preferably within a maximum of 30 minutes, in such a way that the distribution in the patient's body can be detected with spatial resolution.

Fig. 1

Fig. 2

Fig. 3

T-Ninja          T-Ninja-S          T-Ninja-R

Fig. 4

Fig. 5

Fig. 6

Fig. 7

Fig. 8

Fig. 9

Fig. 10

Europäisches Patentamt
European Patent Office
Office européen des brevets

Application Number

EP 23 21 3284

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MORTENSEN M W ET AL: "Functionalization and Cellular Uptake of Boron Carbide Nanoparticles. The First Step toward T Cell-Guided Boron Neutron Capture Therapy", BIOCONJUGATE CHEMISTRY, vol. 17, no. 2, March 2006 (2006-03), pages 284-290, XP055005267, ISSN: 1043-1802, DOI: 10.1021/bc050206v * the whole document * | 1-15 | INV. A61K51/12 A61P35/00 ADD. A61K39/00 C12N5/0783 |
| A | CHEN H-H ET AL: "Regression of recurrent glioblastoma after boron neutron capture therapy and chimeric antigen receptor T-cell therapy in a child", NEURO-ONCOLOGY, vol. 22, no. Suppl. 3, HGC-05, December 2020 (2020-12), page iii345, XP093148827, 19th International Symposium on Pediatric Neuro-Oncology; Nagano, Japan; 13-16 December 2020 ISSN: 1523-5866, DOI: 10.1093/neuonc/noaa222 * abstract * | 1-15 | |
| A | SHI B ET AL: "Multifunctional theranostic nanoparticles for multi-modal imaging-guided CAR-T immunotherapy and chemo-photothermal combinational therapy of non-Hodgkin's lymphoma", BIOMATERIALS SCIENCE, vol. 10, no. 10, 17 May 2022 (2022-05-17), pages 2577-2589, XP093148961, ISSN: 2047-4830, DOI: 10.1039/D1BM01982A | 1-15 | TECHNICAL FIELDS SEARCHED (IPC) A61K A61P C12N |

-/--

The present search report has been drawn up for all claims

| Place of search | Date of completion of the search | Examiner |
|---|---|---|
| The Hague | 10 April 2024 | Teyssier, Bertrand |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,P | SENEVIRATNE D S ET AL: "Next-Generation Boron Drugs and Rational Translational Studies Driving the Revival of BNCT", CELLS, vol. 12, no. 10, 12101398, 16 May 2023 (2023-05-16), XP093148847, ISSN: 2073-4409, DOI: 10.3390/cells12101398 * page 13 - page 14 * | 1-15 | |
| A,P | MONTI HUGHES A ET AL: "Optimizing Boron Neutron Capture Therapy (BNCT) to Treat Cancer: An Updated Review on the Latest Developments on Boron Compounds and Strategies", CANCERS, vol. 15, no. 16, 15164091, 14 August 2023 (2023-08-14), XP093148833, ISSN: 2072-6694, DOI: 10.3390/cancers15164091 * page 21 * | 1-15 | |
| A | WO 2022/066768 A1 (VIRGINIA TECH INTELLECTUAL PROPERTIES INC [US]) 31 March 2022 (2022-03-31) * examples * | 1-15 | TECHNICAL FIELDS SEARCHED (IPC) |
| A | WO 2010/059253 A2 (MASSACHUSETS INST OF TECHNOLOG [US]; IRVINE DARRELL J [US] ET AL.) 27 May 2010 (2010-05-27) * page 29; examples * | 1-15 | |

-/--

The present search report has been drawn up for all claims

| Place of search | Date of completion of the search | Examiner |
|---|---|---|
| The Hague | 10 April 2024 | Teyssier, Bertrand |

EPO FORM 1503 03.82 (P04C01)

Europäisches
Patentamt

European
Patent Office

Office européen
des brevets

## EUROPEAN SEARCH REPORT

Application Number

EP 23 21 3284

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PAI A ET AL: "Dynamically Programmable Magnetic Fields for Controlled Movement of Cells Loaded with Iron Oxide Nanoparticles", ACS APPLIED BIO MATERIALS, vol. 3, no. 7, 28 May 2020 (2020-05-28), pages 4139-4147, XP093011356, ISSN: 2576-6422, DOI: 10.1021/acsabm.0c00226 ----- | 1-15 | |

TECHNICAL FIELDS
SEARCHED       (IPC)

The present search report has been drawn up for all claims

| Place of search | Date of completion of the search | Examiner |
|---|---|---|
| The Hague | 10 April 2024 | Teyssier, Bertrand |

EPO FORM 1503 03.82 (P04C01)

CATEGORY OF CITED DOCUMENTS

X : particularly relevant if taken alone
Y : particularly relevant if combined with another
      document of the same category
A : technological background
O : non-written disclosure
P : intermediate document

T : theory or principle underlying the invention
E : earlier patent document, but published on, or
      after the filing date
D : document cited in the application
L : document cited for other reasons
.......................................................................................
& : member of the same patent family, corresponding
      document

page 3 of 3

## ANNEX TO THE EUROPEAN SEARCH REPORT
## ON EUROPEAN PATENT APPLICATION NO.

EP 23 21 3284

This annex lists the patent family members relating to the patent documents cited in the above-mentioned European search report.
The members are as contained in the European Patent Office EDP file on
The European Patent Office is in no way liable for these particulars which are merely given for the purpose of information.

10-04-2024

| Patent document cited in search report | Publication date | Patent family member(s) | Publication date |
|---|---|---|---|
| WO 2022066768 A1 | 31-03-2022 | US 2023355968 A1 | 09-11-2023 |
| | | WO 2022066768 A1 | 31-03-2022 |
| WO 2010059253 A2 | 27-05-2010 | EP 2398466 A2 | 28-12-2011 |
| | | EP 3936122 A1 | 12-01-2022 |
| | | ES 2866623 T3 | 19-10-2021 |
| | | US 2011293705 A1 | 01-12-2011 |
| | | US 2014017170 A1 | 16-01-2014 |
| | | US 2016256386 A1 | 08-09-2016 |
| | | US 2018110733 A1 | 26-04-2018 |
| | | US 2021259968 A1 | 26-08-2021 |
| | | WO 2010059253 A2 | 27-05-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9855298 B2
- WO 2022241151 A2
- WO 2022238962 A1
- WO 2022241036 A1
- WO 2022147014 A2
- WO 2020109953 A1
- WO 2019181018 A1
- WO 9817342 A2
- CN 115215851 A
- CN 115232129 A
- FR 3120630 A1
- CN 112675442 A
- JP 2016159107 A
- WO 2016005752 A1

**Non-patent literature cited in the description**

- **MIRZAEI et al.** *Prospects for chimeric antigen receptor (CAR) T cells: A potential game changer for adoptive T cell cancer immunotherapy,* 2016
- **ZENG, JIEMING et al.** *Derivation of mimetic T cells endowed with cancer recognition receptors from reprogrammed T cell,* May 2019
- **ROZENBAUM, MEIR et al.** *Gamma-Delta CAR-T Cells Show CAR-Directed and Independent Activity Against Leukemia,* July 2020
- **CARON et al.** *How to Train Your Dragon: Harnessing Gamma Delta T Cells Antiviral Functions and Trained Immunity in a Pandemic Era,* March 2021
- **HANDGRETINGER et al.** *The potential role of T cells after allogeneic HCT for leukemia,* March 2018
- **MALOUFF et al.** *Boron Neutron Capture Therapy: A Review of Clinical Applications,* February 2021